# EUROPEAN PATENT SPECIFICATION

(11) **EP 1 884 240 B1**
(45) Date of publication and mention of the grant of the patent: **11.11.2009**
(21) Application number: 06732194.3
(22) Date of filing: 21.04.2006
(51) Int. Cl.: A61K 31/4178, A61K 31/4164, A61P 1/02, A61P 5/18, A61P 17/00, A61P 19/02, A61P 19/08, A61P 19/10, A61P 29/00, A61P 35/00, C07D 403/10

(54) **PHARMACEUTICAL FOR PREVENTION OR TREATMENT OF BONE METABOLIC DISEASE**
PHARMAZEUTIKUM ZUR PRÄVENTION ODER BEHANDLUNG VON METABOLISCHER KNOCHENKRANKHEIT
PRODUIT PHARMACEUTIQUE POUR LA PRÉVENTION OU LE TRAITEMENT D'UNE MALADIE MÉTABOLIQUE OSSEUSE

(30) Priority: 22.04.2005 JP 2005124374
(43) Date of publication of application: 06.02.2008
(73) Proprietor: Daiichi Sankyo Company, Limited, Tokyo 103-8426 (JP); OSAKA UNIVERSITY, Suita-shi, Osaka 565-0871 (JP)
(72) Inventor: MORISHITA, Ryuuichi, Osaka University, Suita-shi, Osaka, 565-0871 (JP); NAKAGAMI, Hironori, Osaka University, Suita-shi, Osaka, 565-0871 (JP); SHIMIZU, Hideo, Osaka University, Suita-shi, Osaka, 565-0871 (JP)
(74) Representative: Gibson, Christian John Robert
(86) International application number: PCT/JP2006/308386
(87) International publication number: WO 2006/115187

(56) References cited:
- EP-A- 0 503 785
- JP-A- 8 003 044
- JP-A- 05 078 328
- JP-A- 08 003 044
- HAGIWARA, H ET AL: "Deceleration by angiotensin II of the differentiation and bone formation of rat clavarial osteoblastic cells" J ENDOCRINOL, vol. 156, 1998, pages 543-550, XP002487049
- YOSHITAKE H. ET AL.: 'Osteopontin-deficient mice are resistant to ovariectomy-induced bone resorption' PROC. NATL. ACAD. SCI. USA vol. 96, 1999, pages 8156 - 8160, XP003000275
- KAWANO H. ET AL.: 'Angiotensin II enhances integrin and alpha-actinin expression in adult rat cardiac fibroblasts' HYPERTENSION vol. 35, 2000, pages 273 - 279, XP003000276

## Description

### TECHNICAL FIELD

The present invention relates to a medicament for preventing or treating bone metabolic diseases, comprising an angiotensin II receptor antagonist as an active ingredient.

### BACKGROUND ART

Bone metabolic diseases, such as osteoporosis, are increasing steadily in the modem highly aged society. The balance between bone resorption and bone formation is important in many metabolic diseases. While bone resorption by osteoclasts activates osteoblasts and stimulates the osteogenic mechanism, osteoclast activation is mediated by osteoblasts for the most part. A signal transduction system involving these osteoblasts and osteoclasts has been a target for years in the development of various drugs.

Use of an angiotensin II receptor antagonist for the purpose of preventing or treating osteoporosis has been known. For example, Japanese Unexamined Patent Publication No. 8-3044 discloses such use. However, no example of using specific angiotensin II receptor antagonists, such as olmesartan medoxomil, has been known yet. Patent Document No. 1: Japanese Unexamined Patent Publication No. 8-3044

### DISCLOSURE OF THE INVENTION

### PROBLEM FOR SOLUTION BY THE INVENTION

It is an object of the present invention to provide a medicament useful in the prevention or treatment of bone metabolic diseases.

### MEANS TO SOLVE THE PROBLEM

As a result of intensive and extensive researches toward the solution of the above-described problem, the present inventors have found that specific angiotensin II receptor antagonists, such as olmesartan medoxomil, are highly effective in the prevention or treatment of bone metabolic diseases such as osteoporosis. The present invention has been achieved based on this finding.

The present invention provides a medicament for preventing or treating bone metabolic diseases, comprising a specific angiotensin II receptor antagonist as an active ingredient. According to a preferred embodiment of the present invention, a medicament for preventing or treating osteoporosis, rheumatoid arthritis, Paget's disease, bone metastasis of malignant tumor, osteoarthritis, , periodontal disease, dental leakage or alveolar ridge resorption after tooth extraction is provided.

### EFFECT OF THE INVENTION

According to the present invention, it becomes possible to provide a medicament useful in the prevention or treatment of bone metabolic diseases.

The present specification encompasses the contents described in the specification and/or drawings of Japanese Patent Application No. 2005-124374 based on which the present patent application claims priority.

### BRIEF DESCRIPTION OF THE DRAWINGS

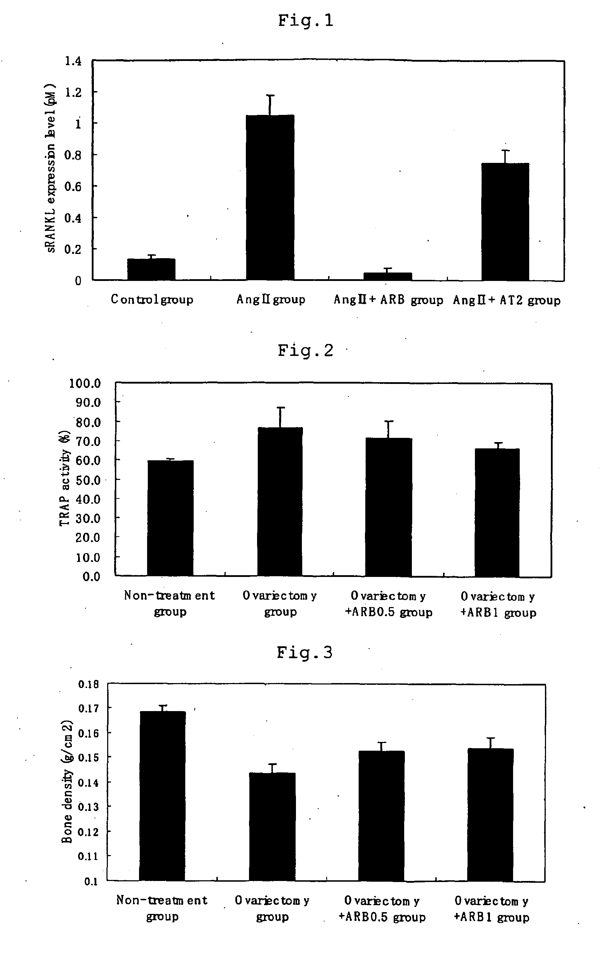

Fig. 1 shows sRANKL expression levels (pM) in human osteoblasts. Control group: non-addition group; AngII group: AngII-added group; AngII+ARB group: AngII and olmesartan were added simultaneously; AngII+AT2 group; AngII and angiotensin type 2 receptor antagonist PD (123319) were added simultaneously.
Fig. 2 shows TRAP activity (%) in 12-week-old female spontaneously hypertensive rats. Non-treatment group: female spontaneously hypertensive rat group where ovariectomy was not performed; Ovariectomy group: female spontaneously hypertensive rat group where ovariectomy was performed at 8 weeks of age; Ovariectomy+ARB0.5 group: female spontaneously hypertensive rat group where olmesartan was administered at 0.5 mg/kg/day with an osmotic pressure pump after ovariectomy was performed at 8 weeks of age; Ovariectomy+ARB 1 group: female spontaneously hypertensive rat group where olmesartan was administered at 1 mg/kg/day with an osmotic pressure pump after ovariectomy was performed at 8 weeks of age.
Fig. 3 shows bone density (g/cm²) in 12-week-old female spontaneously hypertensive rats. Non-treatment group: female spontaneously hypertensive rat group where ovariectomy was not performed; Ovariectomy group: female spontaneously hypertensive rat group where ovariectomy was performed at 8 weeks of age; Ovariectomy+ARB0.5 group: female spontaneously hypertensive rat group where olmesartan was administered at 0.5 mg/kg/day with an osmotic pressure pump after ovariectomy was performed at 8 weeks of age; Ovariectomy+ARB 1 group: female spontaneously hypertensive rat group where olmesartan was administered at 1 mg/kg/day with an osmotic pressure pump after ovariectomy was performed at 8 weeks of age.

### BEST MODE FOR CARRYING OUT THE INVENTION

The angiotensin II receptor antagonist, which is the active ingredient of the present invention, is a compound represented by the following general formula (I), a pharmacologically acceptable salt thereof, or a pharmacologically acceptable ester thereof.

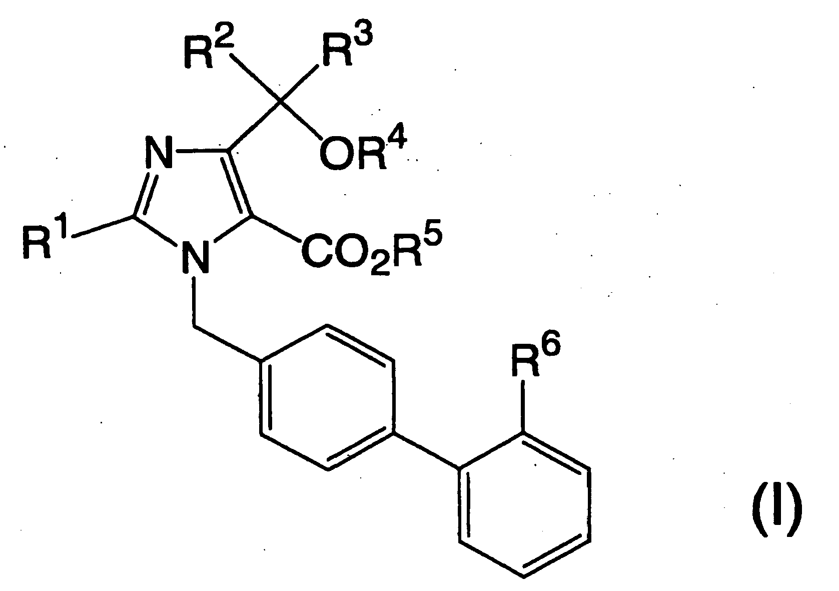

In the above formula,
R¹ represents a C₁-C₄ alkyl group;
R² and R³ are the same or different and each represent a hydrogen atom or a C₁-C₄ alkyl group;
R⁴ represents a hydrogen atom or a C₁-C₄ alkyl group;
R⁵ represents a hydrogen atom, a C₁-C₄ alkyl group, a C₂-C₅ alkanoyloxymethyl or 1-(C₂-C₅ alkanoyloxy)ethyl group, a C₁-C₄ alkoxycarbonyloxymethyl or 1-(C₁-C₄ alkoxycarbonyloxy)ethyl group, a (5-methyl-2-oxo-1,3-dioxolen-4-yl)methyl group, a (5-phenyl-2-oxo-1,3-dioxolen-4-yl)methyl or a phthalidyl group; and
R⁶ represents a carboxy group or a tetrazole-5-yl group.

The C₁-C₄ alkyl group in R¹, R², R³ and R⁴ may be, for example, methyl, ethyl, propyl, isopropyl, butyl or isobutyl group. The C₁-C₄ alkyl group in R¹ is preferably an ethyl, propyl or butyl group, more preferably a propyl or butyl group, and especially preferably a propyl group. The C₁-C₄ alkyl group in R² and R³ is preferably a methyl or ethyl group, and especially preferably a methyl group. The C₁-C₄ alkyl group in R⁴ is preferably a hydrogen atom or methyl group, and especially preferably a hydrogen atom.

R⁵ may be, for example, a hydrogen atom; the above-described C₁-C₄ alkyl group; a C₂-C₅ alkanoyloxymethyl or 1-(C₂-C₅ alkanoyloxy)ethyl group (where the C₂-C₅ alkanoyl moiety may be, for example, acetyl, propionyl, butyryl, isobutyryl, valeryl, isovaleryl or pivaloyl, preferably acetyl or pivaloyl, and especially preferably pivaloyl); a C₁-C₄ alkoxycarbonyloxymethyl or 1-(C₁-C₄ alkoxycarbonyloxy)ethyl group (where the C₁-C₄ alkoxy moiety may be, for example, methoxy, ethoxy, propoxy, isopropoxy, butoxy or isobutoxy, preferably methoxy, ethoxy, propoxy or isopropoxy, and especially preferably ethoxy or isopropoxy); a (5-methyl-2-oxo-1,3-dioxolen-4-yl)methyl group; a (5-phenyl-2-oxo-1,3-dioxolen-4-yl)methyl or a phthalidyl group. Preferably, R⁵ is a methyl group, an ethyl group, an acetoxymethyl group, a 1-(acetoxy)ethyl group, a pivaloyloxymethyl group, a 1-(pivaloyloxy)ethyl group, a methoxycarbonyloxymethyl group, a 1-(methoxycarbonyloxy)ethyl group, an ethoxycarbonyloxymethyl group, a 1-(ethoxycarbonyloxy)ethyl group, a propoxycarbonyloxymethyl group, a 1-(propoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group. Especially preferably, R⁵ is a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group. Most preferably, R⁵ is a (5-methyl-2-oxo-1,3-dioxolen-4-yl)methyl group.

The compound represented by general formula (I) of the present invention may be, if desired, converted to a corresponding pharmacologically acceptable salt by treating with acid or base according to conventional methods. Such a "pharmacologically acceptable salt" may be, for example, an alkaline metal salt such as a sodium salt, potassium salt or lithium salt; an alkaline earth metal salt such as a calcium salt or magnesium salt; a metal salt such as an aluminium salt, iron salt, zinc salt, copper salt, nickel salt or cobalt salt; or an amine salt such as an ammonium salt, t-octylamine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt or tris(hydroxymethyl)aminomethane salt. The pharmacologically acceptable salt is preferably an alkali metal salt, and especially preferably a sodium salt.

The compound represented by general formula (I) of the invention may be converted to a pharmacologically acceptable ester according to conventional methods. The types of the "pharmacologically acceptable ester" are not particularly limited. Any type of ester may be used as long as it has the same pharmaceutical applicability as the compound represented by general formula (I) and is pharmacologically acceptable. For example, a C₁-C₄ alkoxy C₁-C₄ alkyl group such as methoxymethyl, 1-ethoxyethyl, 1-methyl-1-methoxyethyl, 1-(isopropoxy)ethyl, 2-methoxyethyl, 2-ethoxyethyl, 1,1-dimethyl-1-methoxymethyl, ethoxymethyl, propoxymethyl, isopropoxymethyl, butoxymethyl or t-butoxymethyl; a C₁-C₄ alkoxylated C₁-C₄ alkoxy C₁-C₄ alkyl group such as 2-methoxyethoxymethyl; a C₆-C₁₀ aryloxy C₁-C₄ alkyl group such as phenoxymethyl; a halogenated C₁-C₄ alkoxy C₁-C₄ alkyl group such as 2,2,2-trichloroethoxymethyl or bis(2-chloroethoxy)methyl; a C₁-C₄ alkoxycarbonyl C₁-C₄ alkyl group such as methoxycarbonylmethyl; a cyano C₁-C₄ alkyl group such as cyanomethyl or 2-cyanoethyl; a C₁-C₄ alkylthiomethyl group such as methylthiomethyl or ethylthiomethyl; a C₆-C₁₀ arylthiomethyl group such as phenylthiomethyl or naphthylthiomethyl; a C₁-C₄ alkylsulfonyl C₁-C₄ lower alkyl group which may be substituted with a halogen atom(s), such as 2-methanesulfonylethyl or 2-trifluoromethanesulfonylethyl; a C₆-C₁₀ arylsulfonyl C₁-C₄ alkyl group such as 2-benzenesulfonylethyl or 2-toluenesulfonylethyl; a C₁-C₇ aliphatic acyloxy C₁-C₄ alkyl group such as formyloxymethyl, acetoxymethyl, propionyloxymethyl, butyryloxymethyl, pivaloyloxymethyl, valeryloxymethyl, isovaleryloxymethyl, hexanoyloxymethyl, 1-formyloxyethyl, 1-acetoxyethyl, 1-propionyloxyethyl, 1-butyryloxyethyl, 1-pivaloyloxyethyl, 1-valeryloxyethyl, 1-isovaleryloxyethyl, 1-hexanoyloxyethyl, 2-formyloxyethyl, 2-acetoxyethyl, 2-propionyloxyethyl, 2-butyryloxyethyl, 2-pivaloyloxyethyl, 2-valeryloxyethyl, 2-isovaleryloxyethyl, 2-hexanoyloxyethyl, 1-formyloxypropyl, 1-acetoxypropyl, 1-propionyloxypropyl, 1-butyryloxypropyl, 1-pivaloyloxypropyl, 1-valeryloxypropyl, 1-isovaleryloxypropyl, 1-hexanoyloxypropyl, 1-acetoxybutyl, 1-propionyloxybutyl, 1-butyryloxybutyl, 1-pivaloyloxybutyl, 1-acetoxypentyl, 1-propionyloxypentyl, 1-butyryloxypentyl, 1-pivaloyloxypentyl or 1-pivaloyloxyhexyl; a C₅-C₆ cycloalkylcarbonyloxy C₁-C₄ alkyl group such as cyclopentylcarbonyloxymethyl, cyclohexylcarbonyloxymethyl, 1-cyclopentylcarbonyloxyethyl, 1-cyclohexylcarbonyloxyethyl, 1-cyclopentylcarbonyloxypropyl, 1-cyclohexylcarbonyloxypropyl, 1-cyclopentylcarbonyloxybutyl or 1-cyclohexylcarbonyloxybutyl; a C₆-C₁₀ arylcarbonyloxy C₁-C₄ alkyl group such as benzoyloxymethyl; a C₁-C₆ alkoxycarbonyloxy C₁-C₄ alkyl group such as methoxycarbonyloxymethyl, 1-(methoxycarbonyloxy)ethyl, 1-(methoxycarbonyloxy)propyl, 1-(methoxycarbonyloxy)butyl, 1-(methoxycarbonyloxy)pentyl, 1-(methoxycarbonyloxy)hexyl, ethoxycarbonyloxymethyl, 1-(ethoxycarbonyloxy)ethyl, 1-(ethoxycarbonyloxy)propyl, 1-(ethoxycarbonyloxy)butyl, 1-(ethoxycarbonyloxy)pentyl, 1-(ethoxycarbonyloxy)hexyl, propoxycarbonyloxymethyl, 1-(propoxycarbonyloxy)ethyl, 1-(propoxycarbonyloxy)propyl, 1-(propoxycarbonyloxy)butyl, isopropoxycarbonyloxymethyl, 1-(isopropoxycarbonyloxy)ethyl, 1-(isopropoxycarbonyloxy)butyl, butoxycarbonyloxymethyl, 1-(butoxycarbonyloxy)ethyl, 1-(butoxycarbonyloxy)propyl, 1-(butoxycarbonyloxy)butyl, isobutoxycarbonyloxymethyl, 1-(isobutoxycarbonyloxy)ethyl, 1-(isobutoxycarbonyloxy)propyl, 1-(isobutoxycarbonyloxy)butyl, t-butoxycarbonyloxymethyl, 1-(t-butoxycarbonyloxy)ethyl, pentyloxycarbonyloxymethyl, 1-(pentyloxycarbonyloxy)ethyl, 1-(pentyloxycarbonyloxy)propyl, hexyloxycarbonyloxymethyl, 1-(hexyloxycarbonyloxy)ethyl or 1-(hexyloxycarbonyloxy)propyl; a C₅-C₆ cycloalkyloxycarbonyloxy C₁-C₄ alkyl group such as cyclopentyloxycarbonyloxymethyl, 1-(cyclopentyloxycarbonyloxy)ethyl, 1-(cyclopentyloxycarbonyloxy)propyl, 1-(cyclopentyloxycarbonyloxy)butyl, cyclohexyloxycarbonyloxymethyl, 1-(cyclohexyloxycarbonyloxy)ethyl, 1-(cyclohexyloxycarbonyloxy)propyl or 1-(cyclohexyloxycarbonyloxy)butyl; a [5-(C₁-C₄ alkyl)-2-oxo-1,3-dioxolen-4-yl]methyl group such as (5-methyl-2-oxo-1,3-dioxolen-4-yl)methyl, (5-ethyl-2-oxo-1,3-dioxolen-4-yl)methyl, (5-propyl-2-oxo-1,3-dioxolen-4-yl)methyl, (5-isopropyl-2-oxo-1,3-dioxolen-4-yl)methyl or (5-butyl-2-oxo-1,3-dioxolen-4-yl)methyl; a [5-(phenyl which may be substituted with a C₁-C₄ alkyl, C₁-C₄ alkoxy or halogen atom(s))-2-oxo-1,3-dioxolen-4-yl]methyl group such as (5-phenyl-2-oxo-1,3-dioxolen-4-yl)methyl, [5-(4-methylphenyl)-2-oxo-1,3-dioxolen-4-yl]methyl, [5-(4-methoxyphenyl)-2-oxo-1,3-dioxolen-4-yl]methyl, [5-(4-fluorophenyl)-2-oxo-1,3-dioxolen-4-yl]methyl or [5-(4-chlorophenyl)-2-oxo-1,3-dioxolen-4-yl]methyl; or a phthalidyl group which may be substituted with a C₁-C₄ alkyl or C₁-C₄ alkoxy group(s), such as phthalidyl, dimethylphthalidyl or dimethoxyphthalidyl may be used. It should be noted that esters of the compound represented by general formula (I) are not limited to those enumerated above.

A "pharmacologically acceptable salt of a pharmacologically acceptable ester" of the compound (I) of the present invention is a pharmacologically acceptable salt of the above-described "pharmacologically acceptable ester". Such a salt may be, for example, a hydrohalogenic acid salt such as a hydrofluoride, hydrochloride, hydrobromide or hydroiodide; a nitrate; a perchlorate; a sulfate; a phosphate; a C₁-C₄ alkanesulfonic acid salt which may be substituted with a halogen atom(s), such as a methanesulfonate, trifluoromethanesulfonate or ethanesulfonate; a C₆-C₁₀ arylsulfonic acid salt which may be substituted with a C₁-C₄ alkyl group(s), such as a benzenesulfonate or p-toluenesulfonate; a C₁-C₆ aliphatic acid salt such as an acetate, malate, fumarate, succinate, citrate, tartrate, oxalate or maleate; or an amino acid salt such as a glycine salt, lysine salt, arginine salt, ornithine salt, glutamic acid salt or aspartic acid salt, and is preferably a hydrochloride, nitrate, sulfate or phosphate, and is especially preferably a hydrochloride.

When the compound represented by general formula (I) of the present invention (hereinafter, referred to as the "compound (I)") has asymmetric carbon(s) within its molecule, the racemate or optically active substances thereof are also included in the present invention.

The compound (I) and salts thereof, which are the active ingredient of the present invention, may become hydrates as a result of absorption of moisture or attachment of adsorbed water when they have been left in the air. Such salts are also included in the present invention.

The compound (I) and salts thereof, which are the active ingredient of the present invention, may become solvates as a result of absorption of other solvents. Such salts are also included in the present invention.

The compound (I) is preferably:
(1) a compound wherein R¹ is an ethyl group, a propyl group or a butyl group,
(2) a compound wherein R¹ is a propyl group or a butyl group,
(3) a compound wherein R¹ is a propyl group,
(4) a compound wherein R² and R³ are the same or different and each represent a hydrogen atom or a methyl group,
(5) a compound wherein R² and R³ are the same and each represent a methyl group,
(6) a compound wherein R⁴ is a hydrogen atom or a methyl group,
(7) a compound wherein R⁴ is a hydrogen atom,
(8) a compound wherein R⁵ is a hydrogen atom, a methyl group, an ethyl group, an acetoxymethyl group, a 1-(acetoxy)ethyl group, a pivaloyloxymethyl group, a 1-(pivaloyloxy)ethyl group, a methoxycarbonyloxymethyl group, a 1-(methoxycarbonyloxy)ethyl group, an ethoxycarbonyloxymethyl group, a 1-(ethoxycarbonyloxy)ethyl group, a propoxycarbonyloxymethyl group, a 1-(propoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group,
(9) a compound wherein R⁵ is a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group,
(10) a compound wherein R⁵ is a hydrogen atom or a (5-methyl-2-oxo-1,3-dioxolen-4-yl)methyl group, or
(11) a compound wherein R⁶ is a tetrazole-5-yl group. It should be noted here that among groups representing the same group such as R¹ or R², the greater the group number is, the greater the degree of preferability is (e.g., among R¹ groups, (1) is preferable; (2) is more preferable; and (3) is especially preferably).
   Alternatively, a preferable compound may be obtained by selecting R¹ from (1) to (3) described above, selecting R² and R³ from (4) to (5) described above, selecting R⁴ from (6) to (7) described above, selecting R⁵ from (8) to (10) described above, and combining the selected ones or combining the selected ones with R⁶ described in (11) above. For example, the following compounds may be enumerated.
(12) a compound wherein
   R¹ is an ethyl group, a propyl group or a butyl group;
   R² and R³ are the same or different and each represent a hydrogen atom or a methyl group;
   R⁴ is a hydrogen atom or a methyl group; and
   R⁵ is a hydrogen atom, a methyl group, an ethyl group, an acetoxymethyl group, a 1-(acetoxy)ethyl group, a pivaloyloxymethyl group, a 1-(pivaloyloxy)ethyl group, a methoxycarbonyloxymethyl group, a 1-(methoxycarbonyloxy)ethyl group, an ethoxycarbonyloxymethyl group, a 1-(ethoxycarbonyloxy)ethyl group, a propoxycarbonyloxymethyl group, a 1-(propoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group;
(13) a compound wherein
   R¹ is a propyl group or a butyl group;
   R² and R³ are the same and each represent a methyl group;
   R⁴ is a hydrogen atom;
   R⁵ is a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group; and
   R⁶ is a tetrazole-5-yl group;
(14) a compound wherein
   R¹ is a propyl group;
   R² and R³ are the same and each represent a methyl group;
   R⁴ is a hydrogen atom;
   R⁵ is a hydrogen atom or a (5-methyl-2-oxo-1,3-dioxolen-4-yl)methyl group; and
   R⁶ is a tetrazole-5-yl group.

As specific examples of preferable compounds in general formula (I), compounds shown in Table 1 below may be given.

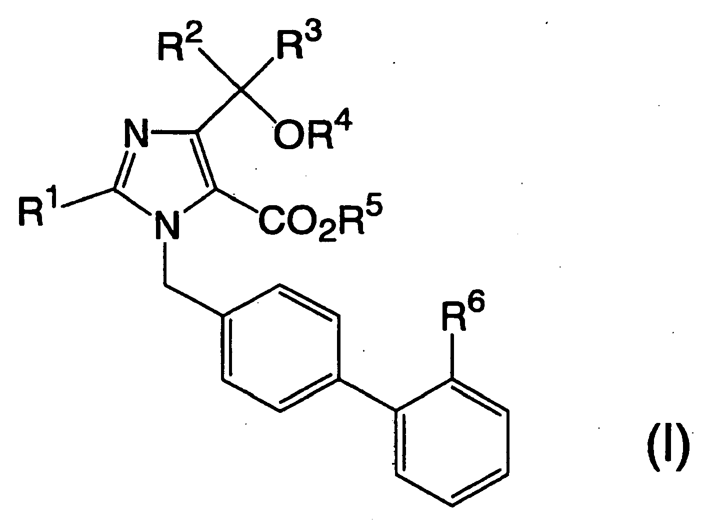

**(Table 1)**

| Compound No. | R¹ | R² | R³ | R⁴ | R⁵ | R⁶ |
|---|---|---|---|---|---|---|
| 1 | Et | Me | Me | H | H | CO₂H |
| 2 | Et | Me | Me | H | Pom | CO₂H |
| 3 | Et | Me | Me | H | Mod | CO₂H |
| 4 | Et | Me | Me | H | H | Tz |
| 5 | Et | Me | Me | H | Pom | Tz |
| 6 | Et | Me | Me | H | Mod | Tz |
| 7 | Pr | H | H | H | H | CO₂H |
| 8 | Pr | H | H | H | Pom | CO₂H |
| 9 | Pr | H | H | H | Mod | CO₂H |
| 10 | Pr | H | H | H | H | Tz |
| 11 | Pr | H | H | H | Pom | Tz |
| 12 | Pr | H | H | H | Mod | Tz |
| 13 | Pr | H | Me | H | H | CO₂H |
| 14 | Pr | H | Me | H | Pom | CO₂H |
| 15 | Pr | H | Me | H | Mod | CO₂H |
| 16 | Pr | H | Me | H | H | Tz |
| 17 | Pr | H | Me | H | Pom | Tz |
| 18 | Pr | H | Me | H | Mod | Tz |
| 19 | Pr | Me | Me | H | H | CO₂H |
| 20 | Pr | Me | Me | H | Me | CO₂H |
| 21 | Pr | Me | Me | H | Et | CO₂H |
| 22 | Pr | Me | Me | H | Pom | CO₂H |
| 23 | Pr | Me | Me | H | CH₂OCO₂Et | CO₂H |
| 24 | Pr | Me | Me | H | CH(Me)OCO₂Et | CO₂H |
| 25 | Pr | Me | Me | H | CH₂OCO₂Prⁱ | CO₂H |
| 26 | Pr | Me | Me | H | CH(Me)OCO₂Prⁱ | CO₂H |
| 27 | Pr | Me | Me | H | Mod | CO₂H |
| 28 | Pr | Me | Me | H | Phth | CO₂H |
| 29 | Pr | Me | Me | H | H | Tz |
| 30 | Pr | Me | Me | H | Me | Tz |
| 31 | Pr | Me | Me | H | Et | Tz |
| 32 | Pr | Me | Me | H | Pom | Tz |
| 33 | Pr | Me | Me | H | CH₂OCO₂Et | Tz |
| 34 | Pr | Me | Me | H | CH(Me)OCO₂Et | Tz |
| 35 | Pr | Me | Me | H | CH₂OCO₂Prⁱ | Tz |
| 36 | Pr | Me | Me | H | CH(Me)OCO₂Prⁱ | Tz |
| 37 | Pr | Me | Me | H | Mod | Tz |
| 38 | Pr | Me | Me | H | Phth | Tz |
| 39 | Pr | Me | Me | Me | H | CO₂H |
| 40 | Pr | Me | Me | Me | Pom | CO₂H |
| 41 | Pr | Me | Me | Me | Mod | CO₂H |
| 42 | Pr | Me | Me | Me | H | Tz |
| 43 | Pr | Me | Me | Me | Pom | Tz |
| 44 | Pr | Me | Me | Me | Mod | Tz |
| 45 | Bu | H | H | H | H | CO₂H |
| 46 | Bu | H | H | H | Pom | CO₂H |
| 47 | Bu | H | H | H | Mod | CO₂H |
| 48 | Bu | H | H | H | H | Tz |
| 49 | Bu | H | H | H | Pom | Tz |
| 50 | Bu | H | H | H | Mod | Tz |
| 51 | Bu | H | Met | H | H | CO₂H |
| 52 | Bu | H | Me | H | Pom | CO₂H |
| 53 | Bu | H | Me | H | Mod | CO₂H |
| 54 | Bu | H | Me | H | H | Tz |
| 55 | Bu | H | Me | H | Pom | Tz |
| 56 | Bu | H | Me | H | Mod | Tz |
| 57 | Bu | Me | Me | H | H | CO₂H |
| 58 | Bu | Me | Me | H | Pom | CO₂H |
| 59 | Bu | Me | Me | H | CH₂OCO₂Et | CO₂H |
| 60 | Bu | Me | Me | H | CH(Me)OCO₂Et | CO₂H |
| 61 | Bu | Me | Me | H | CH₂OCO₂Prⁱ | CO₂H |
| 62 | Bu | Me | Me | H | CH(Me)OCO₂Prⁱ | CO₂H |
| 63 | Bu | Me | Me | H | Mod | CO₂H |
| 64 | Bu | Me | Me | H | Phth | CO₂H |
| 65 | Bu | Me | Me | H | H | Tz |
| 66 | Bu | Me | Me | H | Me | Tz |
| 67 | Bu | Me | Me | H | Et | Tz |
| 68 | Bu | Me | Me | H | Pom | Tz |
| 69 | Bu | Me | Me | H | CH₂OCO₂Et | Tz |
| 70 | Bu | Me | Me | H | CH(Me)OCO₂Et | Tz |
| 71 | Bu | Me | Me | H | CH₂OCO₂Prⁱ | Tz |
| 72 | Bu | Me | Me | H | CH(Me)OCO₂Prⁱ | Tz |
| 73 | Bu | Me | Me | H | Mod | Tz |
| 74 | Bu | Me | Me | H | Phth | Tz |
| 75 | Bu | Me | Me | Me | H | CO₂H |
| 76 | Bu | Me | Me | Me | Pom | CO₂H |
| 77 | Bu | Me | Me | Me | Mod | CO₂H |
| 78 | Bu | Me | Me | Me | H | Tz |
| 79 | Bu | Me | Me | Me | Pom | Tz |
| 80 | Bu | Me | Me | Me | Mod | Tz |

| | | | | | | |
|---|---|---|---|---|---|---|
| Abbreviations used in the above Table indicate the following groups. Bu: butyl group Et: ethyl group Me: methyl group Mod: (5-methyl-2-oxo-1,3-dioxolen-4-yl)methyl group Phth: phthalidyl group Pom: pivaloyloxymethyl group Pr: propyl group Prⁱ: isopropyl group Tz: tetrazole-5-yl group | | | | | | |

In the above Table, preferable compounds are illustrated compounds Nos. 2, 3, 4, 5, 6, 7, 8, 9, 10, 11, 12, 13, 14, 15, 16, 17, 18, 19, 22, 23, 24, 25, 26, 27, 28, 29, 32, 33, 34, 35, 36, 37, 38, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 76, 77, 79 and 80.

More preferable compounds are illustrated compounds Nos. 5, 6, 8, 9, 10, 11, 12, 14, 15, 16, 17, 18, 19, 22, 23, 24, 25, 26, 27, 28, 29, 32, 33, 34, 35, 36, 37, 38, 46, 47, 48, 49, 50, 52, 53, 54, 55, 56, 57, 58, 59, 60, 61, 62, 63, 64, 65, 66, 67, 68, 69, 70, 71, 72, 73 and 74.

Still more preferable compounds are illustrated compounds Nos. 5, 6, 8, 9, 10, 11, 12, 14, 15, 17, 18, 22, 23, 24, 25, 26, 27, 29, 32, 33, 34, 35, 36, 37, 38, 49, 50, 55, 56, 58, 63, 65, 68, 69, 70, 71, 72, 73 and 74.

Especially preferable compounds are:
Illustrated compound No. 11: pivaloyloxymethyl 4-hydroxymethyl-2-propyl-1-[4-[2-(tetrazole-5-yl)phenyl]phenyl]methylimidazole-5-carboxylate,
Illustrated compound No. 12: (5-methyl-2-oxo-1,3-dioxolen-4-yl)methyl 4-hydroxymethyl-2-propyl-1-[4-[2-(tetrazole-5-yl)phenyl]phenyl]methylimidazole-5-carboxylate,
Illustrated compound No. 17: pivaloyloxymethyl 4-(1-hydroxyethyl)-2-propyl -1-[4-[2-(tetrazole-5-yl)phenyl]phenyl]methylimidazole-5-carboxylate,
Illustrated compound No. 18: (5-methyl-2-oxo-1,3-dioxolen-4-yl)methyl 4-(1-hydroxyethyl)-2-propyl-1-[4-[2-(tetrazole-5-yl)phenyl]phenyl]methylimidazole-5-carboxylate,
Illustrated compound No. 29: 4-(1-hydroxy-1-methylethyl)-2-propyl-1-[4-[2-(tetrazole-5-yl)phenyl]phenyl]methylimidazole-5-carboxylic acid,
Illustrated compound No. 32: pivaloyloxymethyl 4-(1-hydroxy-1-methylethyl)-2-propyl-1-[4-[2-(tetrazole-5-yl)phenyl]phenyl]methylimidazole-5-carboxylate,
Illustrated compound No. 33: ethoxycarbonyloxymethyl 4-(1-hydroxy-1-methylethyl)-2-propyl-l-[4-[2-(tetrazole-5-yl)phenyl]phenyl]methylimidazole-5-carboxylate,
Illustrated compound No. 34: 1-(ethoxycarbonyloxy)ethyl 4-(1-hydroxy-1-methylethyl)-2-propyl-1-[4-[2-(tetrazole-5-yl)phenyl]phenyl]methylimidazole-5-carboxylate,
Illustrated compound No. 35: isopropoxycarbonyloxymethyl 4-(1-hydroxy-1-methylethyl)-2-propyl-1-[4-[2-(tetrazole-5-yl)phenyl]phenyl]methylimidazole-5-carboxylate,
Illustrated compound No. 36: 1-(isopropoxycarbonyloxy)ethyl 4-(1-hydroxy-1-methylethyl)-2-propyl-1-[4-[2-(tetrazole-5-yl)phenyl]phenyl]methylimidazole-5-carboxylate,
Illustrated compound No. 37: (5-methyl-2-oxo-1,3-dioxolen-4-yl)methyl 4-(1-hydroxy-1-methylethyl)-2-propyl-1-[4-[2-(tetrazole-5-yl)phenyl]phenyl]methylimidazole-5-carboxylate,
Illustrated compound No. 68: pivaloyloxymethyl 2-butyl-4-(1-hydroxy-1-methylethyl)-1-[4-[2-(tetrazole-5-yl)phenyl]phenyl]methylimidazole-5-carboxylate,
Illustrated compound No. 69: ethoxycarbonyloxymethyl 2-butyl-4-(1-hydroxy-1-methylethyl)-1-[4-[2-(tetrazole-5-yl)phenyl]phenyl]methylimidazole-5-carboxylate,
Illustrated compound No. 70: 1-(ethoxycarbonyloxy)ethyl 2-butyl-4-(1-hydroxy-1-methylethyl)-1-[4-[2-(tetrazole-5-yl)phenyl]phenyl]methylimidazole-5-carboxylate,
Illustrated compound No. 71: isopropoxycarbonyloxymethyl 2-butyl-4-(1-hydroxy -1-methylethyl)-1-[4-[2(tetrazole-5-yl)phenyl]phenyl]methylimidazole-5-carboxylate,
Illustrated compound No. 72: 1-(isopropoxycarbonyloxy)ethyl 2-butyl-4-(1-hydroxy-1-methylethyl)-1-[4-[2-(tetrazole-5-yl)phenyl]phenyl]methylimidazole-5-carboxylate, and
Illustrated compound No. 73: (5-methyl-2-oxo-1,3-dioxolen-4-yl)methyl 2-butyl-4-(1-hydroxy-1-methylethyl)-1-[4-[2-(tetrazole-5-yl)phenyl]phenyl]methylimidazole -5-carboxylate.

Most preferable compounds are:
Illustrated compound No. 29: 4-(1-hydroxy-1-methylethyl)-2-propyl-1-[4-[2-(tetrazole-5-yl)phenyl]phenyl]methylimidazole-5-carboxylic acid (Japanese designation: olmesartan), and
Illustrated compound No. 37: (5-methyl-2-oxo-1,3-dioxolen-4-yl)methyl 4-(1-hydroxy-1-methylethyl)-2-propyl-1-[4-[2-(tetrazole-5-yl)phenyl]phenyl]methylimidazole-5-carboxylate (Japanese designation: olmesartan medoxomil).

Olmesartan medoxomil (illustrated compound No. 37) is a prodrug in which the carboxylic acid at position 5 of the imidazole ring of olmesartan (illustrated compound No. 29) is medoxomil ester. Upon oral administration, this prodrug is hydrolyzed by esterase mainly in the small intestinal epithelium and thus converted to olmesartan, an active substance.

The compound (I) which is the active ingredient of the present invention, pharmacologically acceptable salts thereof and pharmacologically acceptable esters thereof are known (see, for example, Japanese Unexamined Patent Publication No. Hei 5-78328) or may be prepared by known methods (see, for example, Japanese Unexamined Patent Publication No. Hei 5-78328).

The medicament of the present invention may be used in the prevention or treatment of bone metabolic diseases. The term "prevention or treatment" used herein encompasses not only improvement or curing of such diseases but also inhibition of the progress of such diseases, prevention of onset of such diseases, and prevention of recurrence of such diseases. The term "prevention or treatment" should not be interpreted in a limitative manner in any meaning. This term must be interpreted more broadly.

Bone metabolic diseases are diseases of which the major pathology is enhancement of bone resorption by osteoclasts. For example, osteoporosis, rheumatoid arthritis, Paget's disease, bone metastasis of malignant tumor, osteoarthritis, periodontal disease, alveolar pyorrhea, alveolar ridge resorption after tooth extraction and the like are included.

Briefly, the medicament of the present invention comprising a specific angiotensin II receptor antagonist (such as olmesartan medoxomil) as an active ingredient may be used in the prevention or treatment of bone metabolic diseases such as osteoporosis, rheumatoid arthritis, Paget's disease, bone metastasis of malignant tumor, osteoarthritis, , periodontal disease, alveolar pyorrhea, or alveolar ridge resorption after tooth (preferably osteoporosis).

Since the above-described angiotensin II receptor antagonist is generally administered orally, it is desirable to administer the medicament of the present invention orally. However, the administration route of the medicament of the present invention is not limited to oral administration. It may also be administered parenterally, such as intravenously, intrarectally, percutaneously, transmucosally or subcutaneously. Examples of dosage forms suitable for oral administration include, but are not limited to, powder, granules, tablets and capsules. In the preparation of each dosage form, pharmacologically acceptable additives for formulation such as excipients, lubricants, binders, disintegrants, emulsifiers, stabilizers, correctives or diluents may be used appropriately.

As "excipients", for example, organic excipients including sugar derivatives such as lactose, sucrose, glucose, mannitol or sorbitol; starch derivatives such as com starch, potato starch, α-starch or dextrin; cellulose derivatives such as crystalline cellulose; gum arabic; dextran; or pullulan; and inorganic excipients including silicate derivatives such as light anhydrous silicic acid, synthetic aluminium silicate, calcium silicate or magnesium aluminometasilicate; phosphates such as calcium hydrogenphosphate; carbonates such as calcium carbonate; or sulfates such as calcium sulfate may be used.

As "lubricants", for example, stearic acid; metal salts of stearic acid such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate or magnesium lauryl sulfate; silicates such as silicic anhydride and silicic hydrate; or the starch derivatives described above may be used.

As "binders", for example, hydroxypropylcellulose, hydroxypropylmethylcellulose, polyvinylpyrrolidone, macrogol, or compounds similar to the above-described excipients may be used.

As "disintegrants", for example, cellulose derivatives such as low-substituted hydroxypropylcellulose, carboxymethylcellulose, calcium carboxymethylcellulose or internally crosslinked sodium carboxymethylcellulose; crosslinked polyvinylpyrrolidone; and chemically modified starch/cellulose derivatives such as carboxymethylstarch or sodium carboxymethylstarch may be used.

As "emulsifiers", for example, colloidal clay such as bentonite or veegum; metal hydroxides such as magnesium hydroxide or aluminium hydroxide; anionic surfactants such as sodium lauryl sulfate or calcium stearate; cationic surfactants such as benzalkonium chloride; or nonionic surfactants such as polyoxyethylenealkylether, polyoxyethylene sorbitan fatty acid ester or sucrose esters of fatty acids may be used.

As "stabilizers", for example, p-hydroxybenzoate esters such as methylparaben or propylparaben; alcohols such as chlorobutanol, benzyl alcohol or phenylethyl alcohol; benzalkonium chloride; phenols such as phenol or cresol; thimerosal; dehydroacetic acid; or sorbic acid may be used.

As "correctives", for example, sweeteners such as saccharin sodium or aspartame; acidifiers such as citric acid, malic acid or tartaric acid; or flavors such as menthol, lemon or orange may be used.

As "diluents", conventionally used diluents, for example, lactose, mannitol, glucose, sucrose, calcium sulfate, calcium phosphate, hydroxypropylcellulose, microcrystalline cellulose, water, ethanol, polyethylene glycol, propylene glycol, glycerol, starch, polyvinylpyrrolidone, magnesium aluminometasilicate or a mixture of these compounds may be used.

Doses of the medicament of the present invention may be appropriately selected depending on various factors such as the administration route, the type of the active ingredient, the age, body weight or symptoms of the patient, the purpose of administration (prevention or treatment), etc. Generally, the medicament of the present invention is administered at 0.001 mg/kg (preferably 0.01 mg/kg) per day with an upper limit of 10 mg/kg (preferably 1 mg/kg) per day. This dose may be administered once or may be divided into 2 to 6 times a day depending on the symptoms.

The medicament of the present invention may be used in combination with other preparations which are effective for bone metabolic diseases such as osteoporosis, rheumatoid arthritis, Paget's disease, bone metastasis of malignant tumor, osteoarthritis, periodontal disease, alveolar pyorrhea, or alveolar ridge resorption after tooth extraction.

### EXAMPLES

Hereinbelow, the present invention will be described in more detail with reference to the following Examples and Preparation Examples. However, the scope of the present invention is not limited to these Examples and Preparation Examples.

### (Test Example 1)

Purpose: Using a domestic rabbit bone marrow culture system, the number of osteoclasts which were differentiation-induced by active vitamin D3 was measured. Then, the inhibitory effect of olmesartan medoxomil was examined.

Methods: A three-day-old domestic rabbit (New Zealand White Rabbit) was sacrificed, followed by removal of the thigh bone and the tibia. After collection of the bone marrow, the leukocyte (granulocyte-monocyte) fraction was isolated and cultured under conditions of α-MEM, 10% FBS, 5% CO₂, 37°C. 24-well culture plates were used for the culture. The cell count was adjusted to 5 x 10⁴ cells/well. Active vitamin D3 (10⁻⁸ M) was added to the culture system. On day 3, 7 and 10 of the culture, cells were fixed with 4% paraformaldehyde and stained with tartarate-resistant acid phosphatase (TRAP staining) under acidic conditions (pH 5.0). The number of multinucleated cells stained with red (osteoclasts) was measured. Similar measurement was performed with addition of olmesartan at concentrations of 10⁻⁸, 10⁻⁷ and 10⁻⁶ M. With respect to TRAP positive cells (generally, containing 1 to 100 nuclei in one cell), they were divided into three groups depending on the degree of fusion: small cells (containing 1 to 2 nuclei), medium cells (containing 3 to 9 nuclei) and large cells (containing 10 or more nuclei).

Results: As a result of induction using active vitamin D3, in the control group (olmesartan non-addition group), fusion of bone marrow mononuclear cells was observed in the coexistence of mesenchymal cells on day 3; TRAP positive cells were observed sporadically on day 7; and a large number of TRAP positive, multinucleated cells were observed on day 10 (degree of fusion; average cell count: small 101.6, medium 68.6, large 80.6). In olmesartan addition groups, while no remarkable change was recognized in 10⁻⁸ M addition group compared to the control group, a reduction in TRAP positive cell count was recognized on day 10 in 10⁻⁷ M addition group and a significant reduction was recognized in 10⁻⁶ M addition group (small 192.8, medium 50.2, large 23.0). In 10⁻⁶ M addition group, it was also observed that fusion of mononuclear cells observed in the control group on day 3 was significantly reduced. In any of the groups, no remarkable change was observed in the growth of coexisting mesenchymal cells. Although no statistically significant difference was observed in the total number of TRAP positive, multinucleated cells (control group: 250.8; olmesartan addition groups: 265.0), it was observed that fusion into medium or large cells was inhibited in olmesartan addition groups and fusion into small cells was increased. It has been demonstrated that mononuclear TRAP positive cells belonging to the above "small cell" have no bone resorption ability. Therefore, taking all things into consideration, it is judged that osteoclasts contributing to bone resorption are significantly (p<0.05) decreased in olmesartan addition groups (73.2) compared to the control group (149.2).

Discussion: From the above-described experimental results, it is believed that olmesartan and its prodrug olmesartan medoxomil inhibit the induction and formation of osteoclasts in a concentration dependent manner. Further, while olmesartan inhibited the fusion of bone marrow mononuclear cells at effective concentrations, it did not affect the growth of other mesenchymal cells coexisting in the culture system. Therefore, it is believed that this compound specifically inhibits the formation of osteoclasts and that this effect is not caused by cytotoxicity.

Specificity: Based on the above results, it is believed that this compound is effective for bone metabolic diseases (such as osteoporosis, rheumatoid arthritis, Paget's disease, bone metastasis of malignant tumor, osteoarthritis, periodontal disease, alveolar pyorrhea, or alveolar ridge resorption after tooth extraction; preferably osteoporosis) by inhibiting the formation of osteoclasts. In view of the mode of action of this compound, it is believed that its preventive or therapeutic effect will be further enhanced by a combined use with other preparations.

### (Test Example 2)

Purpose: Using human osteoblasts, expression of RANKL (Receptor Activator of NK-κB Ligand) which increases with angiotensin 2 (AngII) was measured. Then, inhibitory effect of olmesartan medoxomil was examined.

Methods: Human osteoblasts (Clonetics Corp., Palo Alto, CA) were cultured in DMEM medium under conditions of 10% FCS, 5% CO₂ and 37°C. AngII (1 µM) (Sigma) was added to the culture. After two-day cultivation, sRANKL (soluble Receptor Activator of NK-κB Ligand) contained in the culture supernatant was examined by EIA assay (Biomedica). Further, the effect produced by simultaneous administration of olmesartan (1 µM) or angiotensin type II receptor antagonist PD (123319) (chemical name: (S)-1-(4-[dimethylamino]-3-methylphenyl)methyl-5-(diphenylacetyl)-4,5,6,7-tetrahydro-1H-imidazo[4,5-c]pyridine-6-carboxylate) (1 µM) (Sigma-aldrich) was also examined.

Results: Significant increase of sRANKL was observed after AngII addition (AngII group: 1.045 ± 0.129 pM) compared to non-addition group (Control group: 0.133 ± 0.0271 pM) (Fig. 1). This increase was almost completely inhibited by simultaneous administration of olmesartan (AngII+ARB group: 0.0442 ± 0.0328 pM), but not by PD (123319) (AngII+AT2 group: 0.747 ± 0.0855 pM).

Discussion: From the results described above, it is believed that AngII increases RANKL expression in osteoblasts to thereby promote the activation of osteoclasts indirectly. On the other hand, it has been found that olmesartan almost completely inhibits this RANKL expression increasing effect of AngII in osteoblasts.

Specificity: Based on the above results, it is believed that olmesartan and its prodrug olmesartan medoxomil are effective for bone metabolic diseases (such as osteoporosis, rheumatoid arthritis, Paget's disease, bone metastasis of malignant tumor, osteoarthritis, periodontal disease, alveolar pyorrhea, or alveolar ridge resorption after tooth extraction; preferably osteoporosis) by inhibiting the activation of osteoclasts by AngII. In view of the mode of action of these compounds, it is believed that their preventive or therapeutic effect will be further enhanced by a combined use with other preparations.

### (Test Example 3)

Purpose: Using spontaneously hypertensive rats, increase in TRAP (tartarate-resistant acid phosphatase) activity in the thigh bone and decrease in the bone density after ovariectomy were measured. Then, inhibitory effects of olmesartan medoxomil against TRAP activity increase and bone density decrease were examined. TRAP is a marker enzyme for osteoclasts, and TRAP activity increases when osteoclasts are activated. When osteoclasts are activated, bone is resorbed and thus bone density is decreased.

Methods: The ovary was removed from 8-week-old female spontaneously hypertensive rats (SHR) (Charles River). After one month observation, the thigh bone was removed, followed by measurement of TRAP activity in the bone (Walter K., et al., Method of Enzymatic Analysis, Academic Press, New York & London: 1974; 856-870) and simultaneous measurement of bone density (Venken K., et al., Bone 2005; 36: 663-670). Further, from immediately after the ovariectomy, olmesartan was administered subcutaneously at two different concentrations of 0.5 mg/kg/day and 1 mg/kg/day using an osmotic pressure pump (Alzet model 2004; Alza Corp).

Results: The TRAP activity in the thigh bone in SHR one month after ovariectomy (Ovariectomy group: 76.9 ± 9.9 U/L) was significantly increased compared to non-treatment group (59.6 ± 1.0 U/L) (Fig. 2). Although administration of olmesartan brought no changes in the length and weight of the bone, concentration dependent decrease was recognized in TRAP activity in the thigh bone (Ovariectomy+ARB0.5 group: 71.7 ± 8.7 U/L; Ovariectomy+ARB 1 group: 66.1 ± 3.6 U/L). Concentration dependent decrease in blood pressure was also recognized as a result of administration of olmesartan (Table A). In the measurement of bone density (dual-energy X-ray absorptiometry), bone density in ovariectomy groups (0.1438 ± 0.003445 g/cm²) was significantly decreased compared to non-treatment group (0.1685 ± 0.002684 g/cm²). However, the administration of olmesartan significantly inhibited the decrease in bone density resulting from ovariectomy (Ovanectomy+ARB0.5group: 0.15273 ± 0.003454 g/cm²; Ovariectomy+ARB1 group: 0.15386 ± 0.004365 g/cm²) (Fig. 3).

**(Table A)**

| Group | Blood Pressure (systolic) | Blood Pressure (diastolic) | Blood Pressure (average) | Heart Rate |
|---|---|---|---|---|
| Non-treatment group (n=3) | 147.667 | 105.667 | 119.333 | 322.333 |
| Ovariectomy group (n=3) | 131.667 | 94.6667 | 107 | 312.667 |
| Ovariectomy+ARB0.5 group | 107 | 68.3333 | 81.3333 | 314 |
| Ovariectomy+ARB1 group (n=3) | 70.8 | 45.8 | 56.25 | 358.6 |

| | | | | |
|---|---|---|---|---|
| The numerical values shown in Table A are average values. | | | | |

Discussion: From the above-described experimental results, it was suggested that olmesartan has inhibitory effect against osteoclast activation even in *in vivo* systems.

Specificity: Based on the above results, it is believed that olmesartan and its prodrug olmesartan medoxomil are effective for bone metabolic diseases (such as osteoporosis, rheumatoid arthritis, Paget's disease, bone metastasis of malignant tumor, osteoarthritis, periodontal disease, alveolar pyorrhea, or alveolar ridge resorption after tooth extraction; preferably osteoporosis) by inhibiting the activation of osteoclasts even in *in vivo* systems. In view of the mode of action of these compounds, it is believed that their preventive or therapeutic effect will be further enhanced by a combined use with other preparations.

### (Preparation Example 1) Capsules

| | |
|---|---|
| Olmesartan medoxomil | 20.0 mg |
| Lactose | 158.7 mg |
| Corn starch | 70.0 mg |
| Magnesium stearate | 1.3 mg |
| Total | 250.0 mg |

The above prescribed powders are mixed and passed through a 60-mesh sieve. The resultant mixture is packed in 250 mg No. 3 gelatin capsules to thereby prepare capsules.

### (Preparation Example 2) Tablets

| | |
|---|---|
| Olmesartan medoxomil | 20.0 mg |
| Lactose | 154.0 mg |
| Com starch | 25.0 mg |
| Magnesium stearate | 1.0 mg |
| Total | 200.0 mg |

The above prescribed powders are mixed and tableted with a tableting machine to thereby prepare 200 mg tablets. These tablets may be coated with sugar, if necessary.

All publications, patents and patent applications cited herein are incorporated herein by reference in their entirety.

### INDUSTRIAL APPLICABILITY

The medicament of the present invention comprising a specific angiotensin II receptor antagonist (such as olmesartan medoxomil) is useful in the prevention or treatment of bone metabolic diseases such as osteoporosis, rheumatoid arthritis, Paget's disease, bone metastasis of malignant tumor, osteoarthritis, periodontal disease, alveolar pyorrhea or alveolar ridge resorption after tooth extraction (preferably osteoporosis). The above-described medicament is preferably for use in warm-blooded animals, and more preferably for use in human.

## Claims

1. Use of a compound represented by the following general formula (I), a pharmacologically acceptable salt thereof or a pharmacologically acceptable ester thereof: 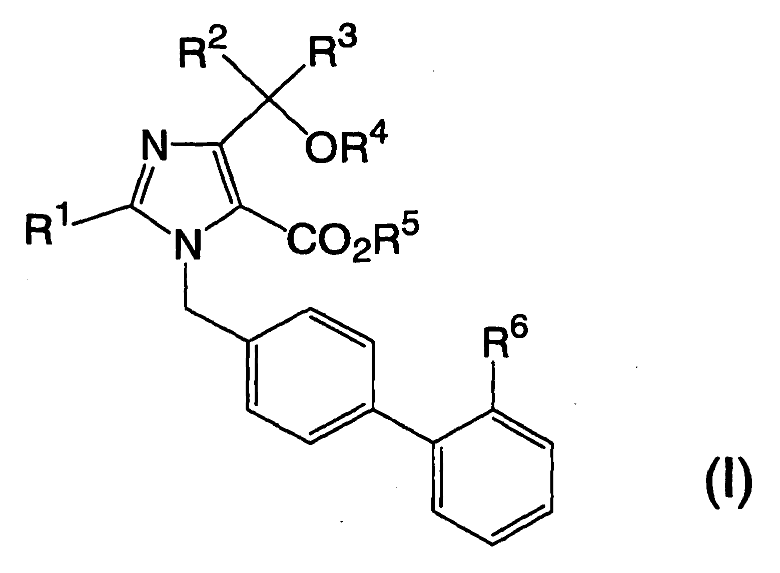 wherein R¹ represents a C₁-C₄ alkyl group;
R² and R³ are the same or different and each represent a hydrogen atom or a C₁-C₄ alkyl group;
R⁴ represents a hydrogen atom or a C₁-C₄ alkyl group;
R⁵ represents a hydrogen atom, a C₁-C₄ alkyl group, a C₂-C₅ alkanoyloxymethyl or 1-(C₂-C₅ alkanoyloxy)ethyl group, a C₁-C₄ alkoxycarbonyloxymethyl or 1-(C₁-C₄ alkoxycarbonyloxy)ethyl group, a (5-methyl-2-oxo-1,3-dioxolen-4-yl)methyl group, a (5-phenyl-2-oxo-1,3-dioxolen-4-yl)methyl or a phthalidyl group; and
R⁶ represents a carboxy group or a tetrazole-5-yl group;
in the manufacture of a medicament for the prophylaxis or therapy of a bone metabolic disease selected from the group consisting of osteoporosis, rheumatoid arthritis, Paget's disease, bone metastasis of malignant tumor, osteoarthritis, periodontal disease, alveolar pyorrhea and alveolar ridge resorption after tooth extraction.

2. Use according to claim 1 of a compound represented by general formula (I) wherein R¹ is an ethyl group, a propyl group or a butyl group, a pharmacologically acceptable salt thereof or a pharmacologically acceptable ester thereof.

3. Use according to claim 1 of a compound represented by general formula (I) wherein R¹ is a propyl group or a butyl group, a pharmacologically acceptable salt thereof or a pharmacologically acceptable ester thereof.

4. Use according to claim 1 of a compound represented by general formula (I) wherein R¹ is a propyl group, a pharmacologically acceptable salt thereof or a pharmacologically acceptable ester thereof.

5. Use according to any one of claims 1 to 4 of a compound represented by general formula (I) wherein R² and R³ are the same or different and each represent a hydrogen atom or a methyl group, a pharmacologically acceptable salt thereof or a pharmacologically acceptable ester thereof.

6. Use according to any one of claims 1 to 4 of a compound represented by general formula (I) wherein R² and R³ are the same and each represent a methyl group, a pharmacologically acceptable salt thereof or a pharmacologically acceptable ester thereof.

7. Use according to any one of claims 1 to 6 of a compound represented by general formula (I) wherein R⁴ is a hydrogen atom or a methyl group, a pharmacologically acceptable salt thereof or a pharmacologically acceptable ester thereof.

8. Use according to any one of claims 1 to 6 of a compound represented by general formula (I) wherein R⁴ is a hydrogen atom, a pharmacologically acceptable salt thereof or a pharmacologically acceptable ester thereof.

9. Use according to any one of claims 1 to 8 of a compound represented by general formula (I) wherein R⁵ is a hydrogen atom, a methyl group, an ethyl group, an acetoxymethyl group, a 1-(acetoxy)ethyl group, a pivaloyloxymethyl group, a 1-(pivaloyloxy)ethyl group, a methoxycarbonyloxymethyl group, a 1-(methoxycarbonyloxy)ethyl group, an ethoxycarbonyloxymethyl group, a 1-(ethoxycarbonyloxy)ethyl group, a propoxycarbonyloxymethyl group, a 1-(propoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group, a pharmacologically acceptable salt thereof or a pharmacologically acceptable ester thereof.

10. Use according to any one of claims 1 to 8 of a compound represented by general formula (I) wherein R⁵ is a hydrogen atom, a pivaloyloxymethyl group, an ethoxycarbonyloxymethyl group, a 1-(ethoxycarbonyloxy)ethyl group, an isopropoxycarbonyloxymethyl group, a 1-(isopropoxycarbonyloxy)ethyl group, a (5-methyl-2-oxo-1,3-dioxolen-4-yl)methyl group or a phthalidyl group, a pharmacologically acceptable salt thereof or a pharmacologically acceptable ester thereof.

11. Use according to any one of claims 1 to 8 of a compound represented by general formula (I) wherein R⁵ is a hydrogen atom or a (5-methyl-2-oxo-1,3-dioxolen-4-yl)methyl group, a pharmacologically acceptable salt thereof or a pharmacologically acceptable ester thereof.

12. Use according to any one of claims 1 to 11 of a compound represented by general formula (I) wherein R⁶ is a tetrazole-5-yl group, a pharmacologically acceptable salt thereof or a pharmacologically acceptable ester thereof.

13. Use according to claim 1 of a compound selected from the group consisting of:
pivaloyloxymethyl 4-hydroxymethyl-2-propyl-1-[4-[2-(tetrazole-5-yl)-phenyl]phenyl]methylimidazole-5-carboxylate,
(5-methyl-2-oxo-1,3-dioxolen-4-yl)methyl 4-hydroxymethyl-2-propyl-1-[4-[2-(tetrazole-5-yl)phenyl]phenyl]methylimidazole-5-carboxylate,
pivaloyloxymethyl 4-(1-hydroxyethyl)-2-propyl-1-[4-[2-(tetrazole-5-yl)phenyl]-phenyl]methylimidazole-5-carboxylate,
(5-methyl-2-oxo-1,3-dioxolen-4-yl)methyl 4-(1-hydroxyethyl)-2-propyl-1-[4-[2-(tetrazole-5-yl)phenyl]phenyl]methylimidazole-5-carboxylate,
4-(1-hydroxy-1-methylethyl)-2-propyl-1-[4-[2-(tetrazole-5-yl)phenyl]phenyl]-methylimidazole-5-carboxylic acid,
pivaloyloxymethyl 4-(1-hydroxy-1-methylethyl)-2-propyl-1-[4-[2-(tetrazole-5-yl)phenyl]phenyl]methylimidazole-5-carboxylate,
ethoxycarbonyloxymethyl 4-(1-hydroxy-1-methylethyl)-2-propyl-1-[4-[2-(tetrazole-5-yl)phenyl]phenyl]methylimidazole-5-carboxylate,
1-(ethoxycarbonyloxy)ethyl 4-(1-hydroxy-1-methylethyl)-2-propyl-1-[4-[2-(tetrazole-5-yl)phenyl]phenyl]methylimidazole-5-carboxylate,
isopropoxycarbonyloxymethyl 4-(1-hydroxy-1-methylethyl)-2-propyl-1-[4-[2-(tetrazole-5-yl)phenyl]phenyl]methylimidazole-5-carboxylate,
1-(isopropoxycarbonyloxy)ethyl 4-(1-hydroxy-1-methylethyl)-2-propyl-1-[4-[2-(tetrazole-5-yl)phenyl]phenyl]methylimidazole-5-carboxylate,
(5-methyl-2-oxo-1,3-dioxolen-4-yl)methyl 4-(1-hydroxy-1-methylethyl)-2-propyl-1-[4-[2-(tetrazole-5-yl)phenyl]phenyl]methylimidazole-5-carboxylate,
pivaloyloxymethyl 2-butyl-4-(1-hydroxy-1-methylethyl)-1-[4-[2-(tetrazole-5-yl)phenyl]phenyl]methylimidazole-5-carboxylate,
ethoxycarbonyloxymethyl 2-butyl-4-(1-hydroxy-1-methylethyl)-1-[4-[2-(tetrazole-5-yl)phenyl]phenyl]methylimidazole-5-carboxylate,
1-(ethoxycarbonyloxy)ethyl 2-butyl-4-(1-hydroxy-1-methylethyl)-1-[4-[2-(tetrazole-5-yl)phenyl]phenyl]methylimidazole-5-carboxylate,
isopropoxycarbonyloxymethyl 2-butyl-4-(1-hydroxy-1-methylethyl)-1-[4-[2-(tetrazole-5-yl)phenyl]phenyl]methylimidazole-5-carboxylate,
1-(isopropoxycarbonyloxy)ethyl 2-butyl-4-(1-hydroxy-1-methylethyl)-1-[4-[2-(tetrazole-5-yl)phenyl]phenyl]methylimidazole-5-carboxylate, and
(5-methyl-2-oxo-1,3-dioxolen-4-yl)methyl 2-butyl-4-(1-hydroxy-1-methylethyl)-1-[4-[2-(tetrazole-5-yl)phenyl]phenyl]methylimidazole-5-carboxylate; a pharmacologically acceptable salt of thereof, or a pharmacologically acceptable ester thereof.

14. Use according to claim 1 of 4-(1-hydroxy-1-methylethyl)-2-propyl-1-[4-[2-(tetrazole-5-yl)phenyl]phenyl]-methylimidazole-5-carboxylic acid or (5-methyl-2-oxo-1,3-dioxolen-4-yl)methyl 4-(1-hydroxy-1-methylethyl)-2-propyl-1-[4-[2-(tetrazole-5-yl)phenyl]phenyl]methylimidazole-5-carboxylate.

15. Use according to claim 1 of 4-(1-hydroxy-1-methylethyl)-2-propyl-1-[4-[2-(tetrazole-5-yl)phenyl]phenyl]methylimidazole-5-carboxylic acid, a pharmacologically acceptable salt of thereof, or a pharmacologically acceptable ester thereof.

16. Use according to any one of claims 1 to 15, wherein the bone metabolic disease is osteoporosis.

17. A compound represented by general formula (I) according to any one of claims 1 to 15, a pharmacologically acceptable salt thereof or a pharmacologically acceptable ester thereof for use in the prophylaxis or therapy of a bone metabolic disease selected from the group consisting of osteoporosis, rheumatoid arthritis, Paget's disease, bone metastasis of malignant tumor, osteoarthritis, periodontal disease, alveolar pyorrhea or alveolar ridge resorption after tooth extraction.

18. A compound for use in the prophylaxis or therapy of bone metabolic diseases according to claim 17, wherein the bone metabolic disease is osteoporosis.

## Patentansprüche

1. Verwendung einer durch die folgende allgemeine Formel (I) dargestellten Verbindung, eines pharmakologisch akzeptierbaren Salzes davon oder eines pharmakologisch akzeptierbaren Esters davon: 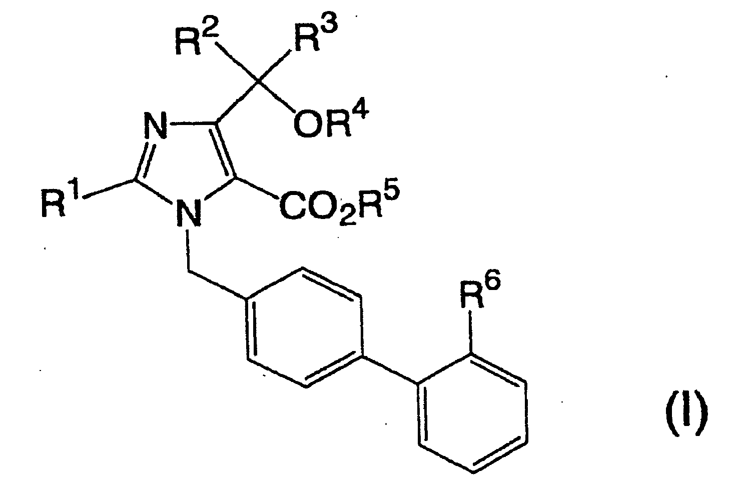 wobei R¹ eine C₁-C₄-Alkylgruppe darstellt;
R² und R³ gleich oder unterschiedlich sind und jeweils ein Wasserstoffatom oder eine C₁-C₄-Alkylgruppe darstellen;
R⁴ ein Wasserstoffatom oder eine C₁-C₄-Alkylgruppe darstellt;
R⁵ ein Wasserstoffatom, eine C₁-C₄-Alkylgruppe, eine C₂-C₅-Alkanoyloxymethyl- oder 1-(C₂-C₅-Alkanoyloxy)ethyl-Gruppe, eine C₁-C₄-Alkoxycarbonyloxymethyl- oder 1-(C₁-C₄-alkoxycarbonyloxy)ethyl-Gruppe, eine (5-Methyl-2-oxo-1,3-dioxolen-4-yl)methyl-Gruppe, eine (5-Phenyl-2-oxo-1,3-dioxolen-4-yl)methyl- oder Phthalidyl-Gruppe darstellt; und
R⁶ eine Carboxygruppe oder Tetrazol-5-yl-Gruppe darstellt;
bei der Herstellung eines Medikaments zur Prophylaxe oder Therapie einer Knochenstoffwechselerkrankung, die aus der Gruppe ausgewählt ist, die aus Osteoporose, rheumatoider Arthritis, Pagetscher Krankheit, einer Knochenmetastase eines bösartigen Tumors, Osteoarthritis, Paradontose, Alveolarpyorrhoe und Alveolarkammresorption nach Zahnextraktion besteht.

2. Verwendung nach Anspruch 1 einer durch die allgemeine Formel (I) dargestellten Verbindung, wobei R¹ eine Ethylgruppe, eine Propylgruppe oder eine Butylgruppe ist, eines pharmakologisch akzeptierbaren Salzes davon oder eines pharmakologisch akzeptierbaren Esters davon.

3. Verwendung nach Anspruch 1 einer durch die allgemeine Formel (I) dargestellten Verbindung, wobei R¹ eine Propylgruppe oder eine Butylgruppe ist, eines pharmakologisch akzeptierbaren Salzes davon oder eines pharmakologisch akzeptierbaren Esters davon.

4. Verwendung nach Anspruch 1 einer durch die allgemeine Formel (I) dargestellten Verbindung, wobei R¹ eine Propylgruppe ist, eines pharmakologisch akzeptierbaren Salzes davon oder eines pharmakologisch akzeptierbaren Esters davon.

5. Verwendung nach einem der Ansprüche 1 bis 4 einer durch die allgemeine Formel (I) dargestellten Verbindung, wobei R² und R³ gleich oder unterschiedlich sind und jeweils ein Wasserstoffatom oder eine Methylgruppe darstellen, eines pharmakologisch akzeptierbaren Salzes davon oder eines pharmakologisch akzeptierbaren Esters davon.

6. Verwendung nach einem der Ansprüche 1 bis 4 einer durch die allgemeine Formel (I) dargestellten Verbindung, wobei R² und R³ gleich oder unterschiedlich sind und jeweils eine Methylgruppe darstellen, eines pharmakologisch akzeptierbaren Salzes davon oder eines pharmakologisch akzeptierbaren Esters davon.

7. Verwendung nach einem der Ansprüche 1 bis 6 einer durch die allgemeine Formel (I) dargestellten Verbindung, wobei R⁴ ein Wasserstoffatom oder eine Methylgruppe ist, eines pharmakologisch akzeptierbaren Salzes davon oder eines pharmakologisch akzeptierbaren Esters davon.

8. Verwendung nach einem der Ansprüche 1 bis 6 einer durch die allgemeine Formel (I) dargestellten Verbindung, wobei R⁴ ein Wasserstoffatom ist, eines pharmakologisch akzeptierbaren Salzes davon oder eines pharmakologisch akzeptierbaren Esters davon.

9. Verwendung nach einem der Ansprüche 1 bis 8 einer durch die allgemeine Formel (I) dargestellten Verbindung, wobei R⁵ ein Wasserstoffatom, eine Methylgruppe, eine Ethylgruppe, eine Acetoxymethylgruppe, eine 1-(Acetoxy)ethyl-Gruppe, eine Pivaloyloxymethyl-Gruppe, eine 1-(Pivaloyloxy)ethyl-Gruppe, eine Methoxycarbonyloxymethyl-Gruppe, eine 1-(Methoxycarbonyloxy)ethylGruppe, eine Ethoxycarbonyloxymethyl-Gruppe, eine 1-(Ethoxycarbonyloxy)ethyl-Gruppe, eine Propoxycarbonyloxymethyl-Gruppe, eine 1-(Propoxycarbonyloxy)ethyl-Gruppe, eine Isopropoxycarbonyloxymethyl-Gruppe, eine 1-(Isopropoxycarbonyloxy)ethyl-Gruppe, eine (5-Methyl-2-oxo-1,3-dioxolen-4-yl)methyl-Gruppe oder eine Phthalidyl-Gruppe ist, eines pharmakologisch akzeptierbaren Salzes davon oder eines pharmakologisch akzeptierbaren Esters davon.

10. Verwendung nach einem der Ansprüche 1 bis 8 einer durch die allgemeine Formel (I) dargestellten Verbindung, wobei R⁵ ein Wasserstoffatom, eine Pivaloyloxymethyl-Gruppe, eine Ethoxycarbonyloxymethyl-Gruppe, eine 1-(Ethoxycarbonyloxy)ethyl-Gruppe, eine Isopropoxycarbonyloxymethyl-Gruppe, eine 1-(Isopropoxycarbonyloxy)ethyl-Gruppe, eine (5-Methyl-2-oxo-1,3-dioxolen-4-yl)methyl-Gruppe oder eine Phthalidyl-Gruppe ist, eines pharmakologisch akzeptierbaren Salzes davon oder eines pharmakologisch akzeptierbaren Esters davon.

11. Verwendung nach einem der Ansprüche 1 bis 8 einer durch die allgemeine Formel (I) dargestellten Verbindung, wobei R⁵ ein Wasserstoffatom oder eine (5-Methyl-2-oxo-1,3-dioxolen-4-yl)methyl-Gruppe ist, eines pharmakologisch akzeptierbaren Salzes davon oder eines pharmakologisch akzeptierbaren Esters davon.

12. Verwendung nach einem der Ansprüche 1 bis 11 einer durch die allgemeine Formel (I) dargestellten Verbindung, wobei R⁶ eine Tetrazol-5-yl-Gruppe ist, eines pharmakologisch akzeptierbaren Salzes davon oder eines pharmakologisch akzeptierbaren Esters davon.

13. Verwendung nach Anspruch 1 einer Verbindung, die aus der Gruppe ausgewählt ist, die aus den folgenden Verbindungen besteht:
Pivaloyloxymethyl-4-hydroxymethyl-2-propyl-1-[4-[2-(tetrazol-5-yl)phenyl]phenyl]methylimidazol-5-carboxylat,
(5-Methyl-2-oxo-1,3-dioxolen-4-yl)methyl-4-hydroxymethyl-2-propyl-1-[4-[2-(tetrazol-5-yl) phenyl]phenyl]methylimidazol-5-carboxylat,
Pivaloyloxymethyl-4-(1-hydroxyethyl)-2-propyl-1-[4-[2-(tetrazol-5-yl)phenyl]phenyl]methylimidazol-5-carboxylat,
(5-Methyl-2-oxo-1,3-dioxolen-4-yl)methyl-4-(1-hydroxyethyl)-2-propyl-1-[4-[2-(tetrazol-5-yl) phenyl]phenyl]methylimidazol-5-carboxylat,
4-(1-Hydroxy-1-methylethyl)-2-propyl-1-[4-[2-(tetrazol-5-yl)-phenyl]phenyl]methylimidazol-5-carbonsäure,
Pivaloyloxymethyl-4-(1-hydroxy-1-methylethyl)-2-propyl-1-[4-[2-(tetrazol-5-yl)phenyl]phenyl] methylimidazol-5-carboxylat,
Ethoxycarbonyloxymethyl-4-(1-hydroxy-1-methylethyl)-2-propyl-1-[4-[2-(tetrazol-5-yl)phenyl] phenyl]methylimidazol-5-carboxylat,
1-(Ethoxycarbonyloxy)ethyl-4-(1-hydroxy-1-methylethyl)-2-propyl-1-[4-[2-(tetrazol-5-yl)phenyl] phenyl]methylimidazol-5-carboxylat,
Isopropoxycarbonyloxymethyl-4-(1-hydroxy-1-methylethyl)-2-propyl-1-[4-[2-(tetrazol-5-yl) phenyl] phenyl]methylimidazol-5-carboxylat,
1-(Isopropoxycarbonyloxy)ethyl-4-(1-hydroxy-1-methylethyl)-2-propyl-1-[4-[2-(tetrazol-5-yl)-phenyl]phenyl]methylimidazol-5-carboxylat,
(5-Methyl-2-oxo-1,3-dioxolen-4-yl)methyl-4-(1-hydroxy-1-methylethyl)-2-propyl-1-[4-[2-(tetrazol-5-yl)phenyl]phenyl]methylimidazol-5-carboxylat,
Pivaloyloxymethyl-2-butyl-4-(1-hydroxy-1-methylethyl)-1-[4-[2-(tetrazol-5-yl)phenyl]phenyl] methylimidazol-5-carboxylat,
Ethoxycarbonyloxymethyl-2-butyl-4-(1-hydroxy-1-methylethyl)-1-[4-[2-(tetrazol-5-yl)phenyl] phenyl]methylimidazol-5-carboxylat,
1-(Ethoxycarbonyloxy)ethyl-2-butyl-4-(1-hydroxy-1-methylethyl)-1-[4-[2-(tetrazol-5-yl)phenyl] phenyl]methylimidazol-5-carboxylat,
Isopropoxycarbonyloxymethyl-2-butyl-4-(1-hydroxy-1-methylethyl)-1-[4-[2-(tetrazol-5-yl)phenyl] phenyl]methylimidazol-5-carboxylat,
1-(Isopropoxycarbonyloxy)ethyl-2-butyl-4-(1-hydroxy-1-methylethyl)-1-[4-[2-(tetrazol-5-yl)-phenyl]phenyl]methylimidazol-5-carboxylat, und
(5-Methyl-2-oxo-1,3-dioxolen-4-yl)methyl-2-butyl-4-(1-hydroxy-1-methylethyl)-1-[4-[2-(tetrazol-5-yl)phenyl]phenyl]methylimidazol-5-carboxylat;
eines pharmakologisch akzeptierbaren Salzes davon oder eines pharmakologisch akzeptierbaren Esters davon.

14. Verwendung nach Anspruch 1 von 4-(1-Hydroxy-1-methylethyl)-2-propyl-1-[4-[2-(tetrazol-5-yl) phenyl]phenyl]methylimidazol-5-carbonsäure oder (5-Methyl-2-oxo-1,3-dioxolen-4-yl)methyl-4-(1-hydroxy-1-methylethyl)-2-propyl-1-[4-[2-(tetrazol-5-yl)-phenyl]phenyl]methylimidazol-5-carboxylat.

15. Verwendung nach Anspruch 1 von 4-(1-Hydroxy-1-methylethyl)-2-propyl-1-[4-[2-(tetrazol-5-yl)-phenyl]phenyl]methylimidazol-5-carbonsäure, einem pharmakologisch akzeptierbaren Salz davon oder einem pharmakologisch akzeptierbaren Ester davon.

16. Verwendung nach einem der Ansprüche 1 bis 15, wobei die Knochenstoffwechselerkrankung Osteoporose ist.

17. Durch die allgemeine Formel (I) dargestellte Verbindung nach einem der Ansprüche 1 bis 15, ein pharmakologisch akzeptierbares Salz davon oder ein pharmakologisch akzeptierbarer Ester davon zur Verwendung bei der Prophylaxe oder Therapie einer Knochenstoffwechselerkrankung, die aus der Gruppe ausgewählt ist, die aus Osteoporose, rheumatoider Arthritis, Pagetscher Krankheit, Knochenmetastase eines bösartigen Tumors, Osteoarthritis, Paradontose, Alveolarpyorrhoe und Alveolarkammresorption nach Zahnextraktion besteht.

18. Verbindung zur Verwendung bei der Prophylaxe oder Therapie von Knochenstoffwechselerkrankungen nach Anspruch 17, wobei die Knochenstoffwechselerkrankung Osteoporose ist.

## Revendications

1. Utilisation d'un composé représenté par la formule générale (I) suivante, d'un sel pharmacologiquement acceptable de celui-ci ou d'un ester pharmacologiquement acceptable de celui-ci: 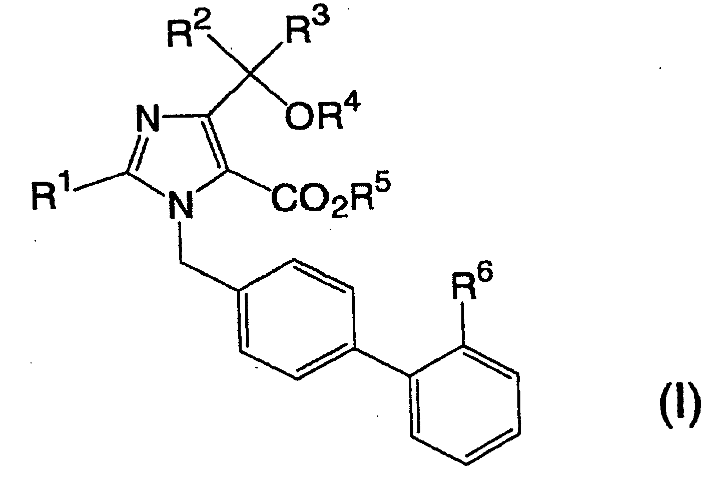 où R¹ représente un groupe alkyle en C₁-C₄;
R² et R³ sont identiques ou différents et chacun représente un atome d'hydrogène ou un groupe alkyle en C₁-C₄;
R⁴ représente un atome d'hydrogène ou un groupe alkyle en C₁-C₄;
R⁵ représente un atome d'hydrogène, un groupe alkyle en C₁-C₄, un groupe alcanoyloxyméthyle en C₂-C₅ ou 1-(alcanoyloxy en C₂-C₅)éthyle, un groupe alcoxycarbonyloxyméthyle en C₁-C₄ ou 1-(alcoxycarbonyloxy en C₁-C₄)éthyle, un groupe (5-méthyl-2-oxo-1,3-dioxolen-4-yl)méthyle, un groupe (5-phényl-2-oxo-1,3-dioxolén-4-yl)méthyle ou phtalidyle; et
R⁶ représente un groupe carboxy ou un groupe tétrazole-5-yle;
dans la fabrication d'un médicament pour la prophylaxie ou la thérapie d'une maladie métabolique osseuse choisie dans le groupe constitué par une ostéoporose, une polyarthrite rhumatoïde, une maladie de Paget, une métastase osseuse de tumeur maligne, une arthrose, une maladie parodontale, une pyorrhée alvéolo-dentaire et une résorption de la crête alvéolaire après extraction de dent.

2. Utilisation selon la revendication 1 d'un composé représenté par la formule générale (I) dans laquelle R¹ est un groupe éthyle, un groupe propyle ou un groupe butyle, d'un sel pharmacologiquement acceptable de celui-ci ou d'un ester pharmacologiquement acceptable de celui-ci.

3. Utilisation selon la revendication 1 d'un composé représenté par la formule générale (I) dans laquelle R¹ est un groupe propyle ou un groupe butyle, d'un sel pharmacologiquement acceptable de celui-ci ou d'un ester pharmacologiquement acceptable de celui-ci.

4. Utilisation selon la revendication 1 d'un composé représenté par la formule générale (I) dans laquelle R¹ est un groupe propyle, d'un sel pharmacologiquement acceptable de celui-ci ou d'un ester pharmacologiquement acceptable de celui-ci.

5. Utilisation selon l'une quelconque des revendications 1 à 4 d'un composé représenté par la formule générale (I) dans laquelle R² et R³ sont identiques ou différents et chacun représente un atome d'hydrogène ou un groupe méthyle, d'un sel pharmacologiquement acceptable de celui-ci ou d'un ester pharmacologiquement acceptable de celui-ci.

6. Utilisation selon l'une quelconque des revendications 1 à 4 d'un composé représenté par la formule générale (I) dans laquelle R² et R³ sont identiques ou différents et chacun représente un groupe méthyle, d'un sel pharmacologiquement acceptable de celui-ci ou d'un ester pharmacologiquement acceptable de celui-ci.

7. Utilisation selon l'une quelconque des revendications 1 à 6 d'un composé représenté par la formule générale (I) dans laquelle R⁴ est un atome d'hydrogène ou un groupe méthyle, d'un sel pharmacologiquement acceptable de celui-ci ou d'un ester pharmacologiquement acceptable de celui-ci.

8. Utilisation selon l'une quelconque des revendications 1 à 6 d'un composé représenté par la formule générale (I) dans laquelle R⁴ est un atome d'hydrogène, d'un sel pharmacologiquement acceptable de celui-ci ou d'un ester pharmacologiquement acceptable de celui-ci.

9. Utilisation selon l'une quelconque des revendications 1 à 8 d'un composé représenté par la formule générale (I) dans laquelle R⁵ est un atome d'hydrogène, un groupe méthyle, un groupe éthyle, un groupe acétoxyméthyle, un groupe 1-(acétoxy)éthyle, un groupe pivaloyloxyméthyle, un groupe 1-(pivaloyloxy)éthyle, un groupe méthoxycarbonyloxyméthyle, un groupe 1-(méthoxycarbonyloxy)éthyle, un groupe éthoxycarbonyloxyméthyle, un groupe 1-(éthoxycarbonyloxy)éthyle, un groupe propoxycarbonyloxyméthyle, un groupe 1-(propoxycarbonyloxy)éthyle, un groupe isopropoxycarbonyloxyméthyle, un groupe 1-(isopropoxycarbonyloxy)éthyle, un groupe (5-méthyl-2-oxo-1,3-dioxolén-4-yl)méthyle ou un groupe phtalidyle, d'un sel pharmacologiquement acceptable de celui-ci ou d'un ester pharmacologiquement acceptable de celui-ci.

10. Utilisation selon l'une quelconque des revendications 1 à 8 d'un composé représenté par la formule générale (I) dans laquelle R⁵ est un atome d'hydrogène, un groupe pivaloyloxyméthyle, un groupe éthoxycarbonyloxyméthyle, un groupe 1-(éthoxycarbonyloxy)éthyle, un groupe isopropoxycarbonyloxyméthyle, un groupe 1-(isopropoxycarbonyloxy)éthyle, un groupe (5-méthyl-2-oxo-1,3-dioxolén-4-yl)méthyle ou un groupe phtalidyle, d'un sel pharmacologiquement acceptable de celui-ci ou d'un ester pharmacologiquement acceptable de celui-ci.

11. Utilisation selon l'une quelconque des revendications 1 à 8 d'un composé représenté par la formule générale (I) dans laquelle R⁵ est un atome d'hydrogène ou un groupe (5-méthyl-2-oxo-1,3-dioxolén-4-yl)méthyle, d'un sel pharmacologiquement acceptable de celui-ci ou d'un ester pharmacologiquement acceptable de celui-ci.

12. Utilisation selon l'une quelconque des revendications 1 à 11 d'un composé représenté par la formule générale (I) dans laquelle R⁶ est un groupe tétrazole-5-yle, d'un sel pharmacologiquement acceptable de celui-ci ou d'un ester pharmacologiquement acceptable de celui-ci.

13. Utilisation selon la revendication 1 d'un composé choisi dans le groupe constitué par:
le 4-hydroxyméthyl-2-propyl-1-[4-[2(-tétrazole-5-yl)-phényl]phényl]méthylimidazole-5-carboxylate de pivaloyloxyméthyle,
le 4-hydroxyméthyl-2-propyl-1-[4-[2(-tétrazole-5-yl)-phényl]phényl]méthylimidazole-5-carboxylate de (5-méthyl-2-oxo-1,3-dioxolén-4-yl)méthyle,
le 4-(1-hydroxyéthyl)-2-propyl-1-[4-[2(-tétrazole-5-yl)-phényl]phényl]méthylimidazole-5-carboxylate de pivaloyloxyméthyle,
le 4-(1-hydroxyéthyl)-2-propyl-1-[4-[2(-tétrazole-5-yl)-phényl]phényl]méthylimidazole-5-carboxylate de (5-méthyl-2-oxo-1,3-dioxolén-4-yl)méthyle,
l'acide 4-(1-hydroxy-1-méthyléthyl)-2-propyl-1-[4-[2-(tétrazole-5-yl)phényl]phényl]-méthylimidazole-5-carboxylique,
le 4-(1-hydroxy-1-méthyléthyl)-2-propyl-1-[4-[2-(tétrazole-5-yl)phényl]phényl]-méthylimidazole-5-carboxylate de pivaloyloxyméthyle,
le 4-(1-hydroxy-1-méthyléthyl)-2-propyl-1-[4-[2-(tétrazole-5-yl)phényl]phényl]-méthylimidazole-5-carboxylate d'éthoxycarbonyloxyméthyle,
le 4-(1-hydroxy-1-méthyléthyl)-2-propyl-1-[4-[2-(tétrazole-5-yl)phényl]phényl]-méthylimidazole-5-carboxylate de 1-(éthoxycarbonyloxy)éthyle,
le 4-(1-hydroxy-1-méthyléthyl)-2-propyl-1-[4-[2-(tétrazole-5-yl)phényl]phényl]-méthylimidazole-5-carboxylate d'isopropoxycarbonyloxyméthyle,
le 4-(1-hydroxy-1-méthyléthyl)-2-propyl-1-[4-[2-(tétrazole-5-yl)phényl]phényl]-méthylimidazole-5-carboxylate de 1-(isopropoxycarbonyloxy)éthyle,
le 4-(1-hydroxy-1-méthyléthyl)-2-propyl-1-[4-[2-(tétrazole-5-yl)phényl]phényl]-méthylimidazole-5-carboxylate de (5-méthyl-2-oxo-1,3-dioxolén-4-yl)méthyle,
le 2-butyl-4-(1-hydroxy-1-méthyléthyl)-1-[4-[2-(tétrazole-5-yl)phényl]phényl]-méthylimidazole-5-carboxylate de pivaloyloxyméthyle,
le 2-butyl-4-(1-hydroxy-1-méthyléthyl)-1-[4-[2-(tétrazole-5-yl)phényl]phényl]-méthylimidazole-5-carboxylate d'éthoxycarbonyloxyméthyle,
le 2-butyl-4-(1-hydroxy-1-méthyléthyl)-1-[4-[2-(tétrazole-5-yl)phényl]phényl]-méthylimidazole-5-carboxylate de 1-(éthoxycarbonyloxy)éthyle,
le 2-butyl-4-(1-hydroxy-1-méthyléthyl)-1-[4-[2-(tétrazole-5-yl)phényl]phényl]-méthylimidazole-5-carboxylate d'isopropoxycarbonyloxyméthyle,
le 2-butyl-4-(1-hydroxy-1-méthyléthyl)-1-[4-[2-(tétrazole-5-yl)phényl]phényl]-méthylimidazole-5-carboxylate de 1-(isopropoxycarbonyloxy)éthyle, et
le 2-butyl-4-(1-hydroxy-1-méthyléthyl)-1-[4-[2-(tétrazole-5-yl)phényl]phényl]-méthylimidazole-5-carboxylate de (5-méthyl-2-oxo-1,3-dioxolén-4-yl)méthyle;
d'un sel pharmacologiquement acceptable de celui-ci ou d'un ester pharmacologiquement acceptable de celui-ci.

14. Utilisation selon la revendication 1 d'acide 4-(1-hydroxy-1-méthyléthyl)-2-propyl-1-[4-[2-(tétrazole-5-yl)phényl]phényl]-méthylimidazole-5-carboxylique ou de 4-(1-hydroxy-1-méthyléthyl)-2-propyl-1-[4-[2-(tétrazole-5-yl)phényl]phényl]-méthylimidazole-5-carboxylate de (5-méthyl-2-oxo-1,3-dioxolén-4-yl)méthyle.

15. Utilisation selon la revendication 1 d'acide 4-(1-hydroxy-1-méthyléthyl)-2-propyl-1-[4-[2-(tétrazole-5-yl)phényl]phényl]-méthylimidazole-5-carboxylique, d'un sel pharmacologiquement acceptable de celui-ci ou d'un ester pharmacologiquement acceptable de celui-ci.

16. Utilisation selon l'une quelconque des revendications 1 à 15, dans laquelle la maladie métabolique osseuse est l'ostéoporose.

17. Composé représenté par la formule générale (I) selon l'une quelconque des revendications 1 à 15, sel pharmacologiquement acceptable de celui-ci ou ester pharmacologiquement acceptable de celui-ci, pour l'utilisation dans la prophylaxie ou la thérapie d'une maladie métabolique osseuse choisie dans le groupe constitué par une ostéoporose, une polyarthrite rhumatoïde, une maladie de Paget, une métastase osseuse de tumeur maligne, une arthrose, une maladie parodontale, une pyorrhée alvéolo-dentaire et une résorption de la crête alvéolaire après extraction de dent.

18. Composé pour l'utilisation dans la prophylaxie ou la thérapie des maladies métaboliques osseuses selon la revendication 17, dans lequel la maladie métabolique osseuse est l'ostéoporose.
